# EUROPEAN PATENT SPECIFICATION

(11) **EP 0 396 412 B1**
(45) Date of publication and mention of the grant of the patent: **13.10.1993**
(21) Application number: 90304812.2
(22) Date of filing: 03.05.1990
(51) Int. Cl.: A01K 67/033, A01N 63/00

(54) **Pest control**
Schädlingsbekämpfung
Lutte contre les parasites

(30) Priority: 04.05.1989 GB 8910233
(43) Date of publication of application: 07.11.1990
(73) Proprietor: BIOLOGICAL CROP PROTECTION LIMITED, West Wittering, Chichester PO20 8QG (GB)
(72) Inventor: Dale, John, Dr., Colchester, Essex (GB)
(74) Representative: Carmichael, David Andrew Halliday

(56) References cited:
- GB-A- 2 000 007
- GB-A- 2 050 836
- GB-A- 2 052 261
- GB-A- 2 168 680
- US-A- 3 786 594

## Description

The present invention relates to pest control in horticulture, and is primarily concerned with a distribution system for biological control agents in glasshouses.

Currently, biological control agents such as parasitic wasp larvae, or pupae of prey insects parasitised by such wasps, are used in glasshouses to control pest insects. Distribution of such agents in the glasshouse is currently achieved by the supplier providing a carrier or sealed container holding a predetermined number of control insects either as larvae or pupae of parasitised pest insects sufficient for a single site, the container or carrier being first opened and then placed at the appropriate location within the glasshouse. In time, the control insects reach a mature stage whereupon they emerge as adult control insects and leave the container in search of pest insects upon which to prey. Generally, containers are supplied on a weekly basis, since the insects emerge from their pupae in a few days.

This system has serious disadvantages both in terms of transportation of containerised control insects and in terms of distribution in the glasshouse. Firstly, the container must provide ample free space for the control insects to emerge from their pre-adult stage, and thus the volume of the container is necessarily many times that of the control insects being transported. A major disadvantage of this transportation system is that, due to the relatively small volume of control insects in relation to the packaging volume, thermal insulation of the insects is difficult and temperature fluctuations inside the package can permit premature emergence of the insects if too high a temperature is reached, or death of the insects if the temperature is too low, with obvious disadvantage to the end user.

Secondly, and more importantly with regard to the efficacy of the control operation, the location at which the container is deployed in the glasshouse depends largely on the assiduity of the glasshouse operator in following the site plan determined for the pest control operation. It has been found that operators will deploy opened containers only in areas easily accessible from walkways in the glasshouse, leaving remoter regions unprotected.

A method according to the preamble of claim 1 is known, see in particular GB-A-2052261.

The present invention seeks to provide a distribution system for control insects, mites or other arthropods which avoids the problems inherent in the prior art.

Pest control in an area of vegetation is achieved by defining a number of fixed release sites in and about the area, and placing at each site a predetermined number of pre-adult control insects at intervals, the control insects being placed at the sites by delivering a fixed volume of pre-adult control insects from a dispenser.

According to the invention as claimed in claim 1, a method of controlling a population of insect pests in an area of vegetation comprises the steps of defining a number of fixed release sites in and about the area, and placing a predetermined number of pre-adult control insects at each release site at predetermined intervals to provide a continuing supply of emerging control insects at each release site, the control insects being placed at the release sites by delivering a predetermined volume of pre-adult insects from a bulk supply using a dispenser, and is characterised by the use of a dispenser comprising a container whose interior is divided to define a storage volume and a measuring volume connected via a transfer opening, the storage volume being so configured that with the dispenser in a first orientation the pre-adult insects in the storage volume are guided towards the transfer opening so as to flow therethrough until the measuring volume is filled, the measuring volume having an outlet passage positioned so as to be inaccessible to the pre-adult insects when the dispenser is in its first orientation, movement of the dispenser to a second orientation causing pre-adult insects remaining in the storage volume to fall away from the transfer opening, and causing the pre-adult insects in the measuring volume to be discharged through the outlet passage.

Preferably the movement required is an inversion of the container. However, rotation of the container about a horizontal axis and through an angle of less than 180° may be sufficient to dispense the material.

In a preferred dispenser for use in the method, there is provided a body of cylindrical shape having an open upper end and its lower end tapered downwardly to a point, a lid closing the open upper end and having a dispensing tube extending axially to contact the lower end wall of the container along a line encircling its point, the tube being provided at its one end with one or more cutouts to afford access between the lumen of the tube and the interior of the container. In such a dispenser, control insects or a mixture of control insects and other granular material such as nutrient, which will hereinafter be referred to as 'pupae' are placed in the container, and the cap and dispenser tube applied. Inverting the container allows one 'dose' of pupae to exit via the dispensing tube, the remainder being retained in the container. Righting the dispenser recharges the dispensing tube with the required volume of pupae for the next 'dose'.

Preferably the dispenser tube is formed with a pair of diametrically opposite cutouts at its one end contacting the conical lower end wall of the container. In such an arrangement, it is preferable to invert the container about an axis passing through both cutouts. This minimises the amount of material which falls back through the cutouts during inversion.

Clearly, when only one such cutout is provided the preferred inversion movement is one which keeps the cutout facing upwardly, to minimise loss of material from the dispensing volume through the cutout.

Preferably the control insects are delivered to the release sites once a week, but more or less frequent delivery is possible. Control insects may be delivered daily, or monthly, or at any interval therebetween. For weekly delivery, about 100 insects are placed at each site during each delivery. Release sites are preferably spaced evenly throughout the infested area, ideally one release site being provided per 100 square metres of area to be covered. In cold weather the release sites may be positioned adjacent heat sources to promote the emergence of the control insects.

In the embodiment of the invention to be described later, pre-adult stages of encarsia are used as the control insects to regulate populations of whitefly in glasshouse crops. Other, alternative, control insects are usable with the method, depending on the pest insect species to be controlled. Examples of suitable control insects are mites, wasps or arthropods. Mites are most easily used in adult or pre-adult stages, wasps are most suitable when in pre-adult stages, and arthropods can be used at any stage of their development.

An embodiment of the invention will now be described with reference to the accompanying drawing, which is a longitudinal sectional view of a dispenser for granular material.

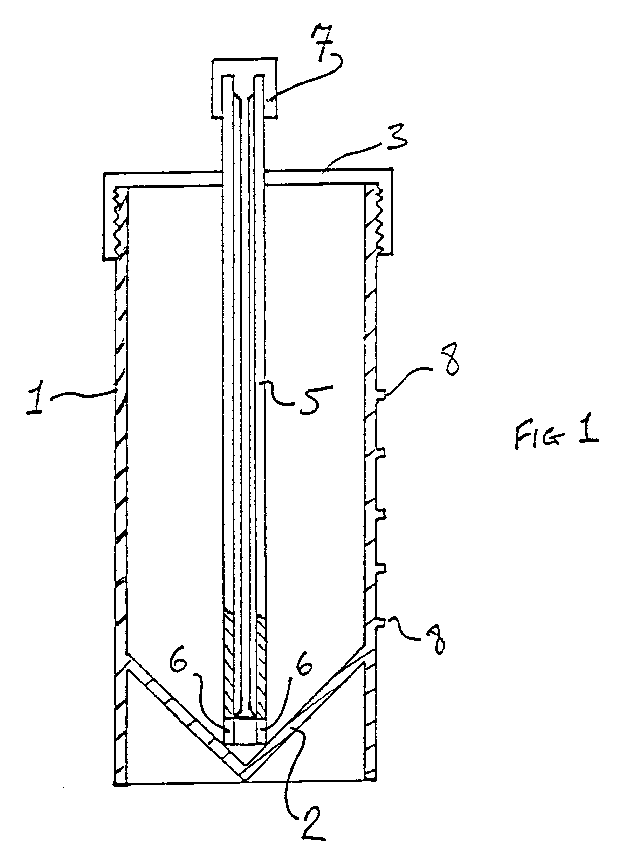

Referring now to Figure 1, there is seen a cylindrical container 1 having a downwardly tapered conical base wall 2. A cap 3 closes the upper end of the container 1, and a dispensing tube 5 extends axially down from the cap 3 to contact the base wall 2. Two diametrically opposed cutouts 6 at the one lower end of the dispensing tube 5 allow access from the interior of container 1 to the lumen of the dispensing tube 5 and constitute a transfer opening, the axial extend of the cutouts 6 being important as will be discussed below. The dispensing tube 5 extends upwardly out of the cap 3, the other upper exit end 4 of the tube 5 being bevelled to form a chute. A closure 7 is arranged to block the dispensing tube 5, preferably extending down the dispenser tube to close it at the upper ends of the cutouts 6.

Operation of the dispenser is simple; pupae are loaded into the container 1 by removing screw threaded cap 3 and filling the container to the required level. Indicia 8 may be provided on the container, calibrated to show the approximate number of pupae required to fill the container to a predetermined level. Alternatively, a label bearing graduations may be applied to the container.

When the pupae are in place, the cap 3 and dispensing tube 5 are replaced. To dispense a single 'dose' of pupae, the closure 7 is removed and the container is simply inverted, whereupon the pupae are discharged by gravity from the tube 5. Preferably the container is inverted about an axis passing through the two cutouts 6, so that the number of pupae falling out of the tube 5 through the cutouts 6 is minimised. The outer end of the tube 5 may be shaped to indicate the correct orientation for dispensing, such as by bevelling the end 4 of the tube 5 to form a 'chute', or by bending the tube adjacent its end about a transverse axis. This will encourage the operator to invert the dispenser correctly, since the end of the dispenser tube will have a perceived preferred orientation.

On righting the container, the conical base wall 2 acts to guide pupae down towards the cutouts 6, whereupon the lower end of the dispenser tube 5 is filled to a predetermined height depending on the axial extent of the cutouts 6. Clearly the volume of the filled part of the tube 5, together with the apex of the conical base wall 2, defines the volume of the dose of pupae to be dispensed. It is understood that inversion of the container with the closure 7 in place will not dispense pupae, but more importantly the blocking of the dispenser tube at the level of the cutouts not only prevents the escape of prematurely emergent insects, but also prevents a build-up of pupae in the dispenser tube if the container is repeatedly inverted in transit.

The conical form of base wall 2 guides the pupae towards the cutouts 6 and ensures that the dispensing tube will be adequately filled even when only a few pupae remain in the container - the entire contents of the container may thus be utilised in regulated doses without wastage.

An advantage of using the dispenser is that the pupae may be shipped in bulk from the producer to the distributor, thus saving transportation cost since the volume of the bulk package is considerably less than is the case when the pupae are pre-packaged in individual release site packs. The pupae may even be refrigerated for longer journeys, for example by using insulated containers, to delay emergence of the insects.

When the bulk pupae reach the distributor, he can then prepare a dispenser containing the appropriate number of pupae for each of his customers, and deliver these to the glasshouse operators preferably in well-insulated containers.

Alternatively, the pupae for a single treatment of a number of release sites may be sent direct to the glasshouse operator in a sealed container, the pupae being transferred on arrival to a dispenser for use in the glasshouse.

## Claims

1. A method of controlling a population of insect pests in an area of vegetation, comprising the steps of defining a number of fixed release sites in and about the area, and placing a predetermined number of pre-adult control insects at each release site at predetermined intervals to provide a continuing supply of emerging control insects at each release site, the control insects being placed at the release sites by delivering a predetermined volume of pre-adult insects from a bulk supply using a dispenser,
characterised by
the use of a dispenser comprising a container whose interior is divided to define a storage volume (1, 2) and a measuring volume (5, 6) connected via a transfer opening (6), the storage volume (1, 2) being so configured that with the dispenser in a first orientation the pre-adult insects in the storage volume are guided towards the transfer opening so as to flow therethrough until the measuring volume (5, 6) is filled, the measuring volume having an outlet passage positioned so as to be inaccessible to the pre-adult insects when the dispenser is in its first orientation, movement of the dispenser to a second orientation causing pre-adult insects remaining in the storage volume to fall away from the transfer opening (6), and causing the pre-adult insects in the measuring volume to be discharged through the outlet passage.

2. A method according to Claim 1 comprising the use of a dispenser, wherein the storage volume (1, 2) is generally cylindrical and has a conical end wall (2) tapering to a point at one end, and the measuring volume is defined by a dispensing tube (5) extending substantially axially of the storage volume, one end of the tube contacting the conical end wall (2) along a line surrounding the point, and the other, exit, end of the tube extending out of the storage volume, the transfer opening (6) being formed in the wall (5) of the tube at its one end contacting the conical end wall (2) of the storage volume to provide access from the storage volume to the lumen of the tube.

3. A method according to Claim 2 comprising the use of a dispenser, wherein the transfer opening (6) comprises a cutout (6) in the one end of the dispensing tube (5).

4. A method according to Claim 3 comprising the use of a dispenser, wherein the transfer opening (6) comprises two diametrically opposed cutouts (6).

5. A method according to Claim 4 comprising the use of a dispenser, wherein the exit end of the dispensing tube (5) remote from the conical end wall (2) of the storage volume terminates in a plane inclined to the longitudinal axis of the storage volume and parallel to the axis passing through the opposed cutouts (6).

6. A method according to Claim 5 comprising the use of a dispenser, wherein the exit end of the dispensing tube (5) is bevelled.

7. A method according to Claim 5 comprising the use of a dispenser, wherein the exit end of the dispensing tube (5) is inclined relative to the axis of the storage volume (1).

## Patentansprüche

1. Verfahren zur Kontrollierung einer Population von Insekten-Schädlingen in einem Vegetationsbereich, das die Schritte des Definierens einer Anzahl von festen Hinauslaß-Stellen in und um den Bereich und des Plazierens einer vorbestimmten Anzahl von vor-erwachsenen Kontrollinsekten an jeder Hinauslaß-Stelle in vorbestimmten Intervallen umfaßt, um eine kontinuierliche Zuführung von hervorkommenden Kontrollinsekten an jeder Hinauslaß-Stelle zu schaffen, wobei die Kontrollinsekten an den Hinauslaß-Stellen durch Lieferung einer vorbestimmten Menge an vor-erwachsenen Insekten aus einer Massenzuführung durch Verwendung eines Verteilers plaziert werden;
gekennzeichnet durch
die Verwendung eines Verteilers, welcher einen Behälter umfaßt, dessen Innenraum geteilt ist, um ein Lagervolumen (1,2) und ein Abmessvolumen (5,6) zu definieren, welche durch eine Übertragungsöffnung (6) miteinander verbunden sind, wobei das Lagervolumen (1,2) derart konfiguriert ist, daß, wenn sich der Verteiler in einer ersten Orientierung befindet, die vorerwachsenen Insekten in dem Lagervolumen zur Übertragungsöffnungen geführt werden, um dort hindurchzufliegen, bis das Abmessvolumen (5,6) gefüllt ist, wobei das Abmessvolumen eine Auslasspassage aufweist, welche derart positioniert ist, daß sie für die vor-erwachsenen Insekten unerreichbar ist, wenn sich der Verteiler in seiner ersten Orientierung befindet, wobei die Bewegung des Verteilers in eine zweite Orientierung die in dem Lagervolumen zurückbleibenden vor-erwachsenen Insekten dazu bringt, von der Übertragungsöffnung (6) wegzufallen und die sich in dem Abmessvolumen befindlichen vor-erwachsenen Insekten dazu bringt, durch die Auslasspassage freigelassen zu werden.

2. Verfahren nach Anspruch 1, welches die Verwendung eines Verteilers umfaßt, worin das Lagervolumen (1,2) im allgemeinen zylindrisch ausgebildet ist und eine konische Endwand (2) aufweist, welche sich zu einem Punkt an einem Ende hin verjüngt, und das Abmessvolumen durch ein Verteilerrohr (5) definiert wird, welches sich im wesentlichen axial zum Lagervolumen erstreckt, dessen eines Ende die konische Endwand (2) entlang einer Linie, welche den Punkt umgibt, kontaktiert und dessen anderes Ausgangsende sich aus dem Lagervolumen hinaus erstreckt, wobei die Übertragungsöffnung (6) in der Wand (5) des Rohres ausgebildet ist und mit ihrem einen Ende die konische Endwand (2) des Lagervolumens kontaktiert, um einen Zugang von dem Lagervolumen zum Lumen des Rohres zu schaffen.

3. Verfahren nach Anspruch 2, welches die Verwendung eines Verteilers umfaßt, worin die Übertragungsöffnung (6) eine Aussparung in dem einen Ende des Verteilerrohres (5) umfaßt.

4. Verfahren nach Anspruch 3, welches die Verwendung eines Verteilers umfaßt, worin die Übertragungsöffnung (6) zwei diametrisch gegenüberliegende Aussparungen umfaßt.

5. Verfahren nach Anspruch 4, welches die Verwendung eines Verteilers umfaßt, worin das von der konischen Endwand (2) des Lagervolumens fernliegende Ausgangsende des Verteilerrohres in einer Ebene endet, welche um die longitudinale Achse des Lagervolumens geneigt und parallel zu der durch die gegenüberliegenden Aussparungen (6) laufenden Achse ist.

6. Verfahren nach Anspruch 5, welches die Verwendung eines Verteilers umfaßt, worin das Ausgangsende des Verteilerrohres (5) abgeflacht ist.

7. Verfahren nach Anspruch 5, welches die Verwendung eines Verteilers umfaßt, worin das Ausgangsende des Verteilerrohres (5) relativ zur Achse des Lagervolumens (1) geneigt ist.

## Revendications

1. Procédé de contrôle d'une population d'insectes nuisibles dans une région de végétation comprenant les étapes consistant à définir un nombre de sites fixes de lâcher à l'intérieur et dans le voisinage de la région, placer un nombre prédéterminé d'insectes de contrôle pré-adultes dans chaque site de lâcher, à des intervalles prédéterminés, pour constituer une réserve permanente d'insectes de contrôle apparaissant dans chaque site de lâcher, les insectes de contrôle étant placés dans les sites de lâcher par la délivrance d'un volume prédéterminé d'insectes pré-adultes à partir d'une réserve en nombre utilisant un distributeur caractérisé par l'utilisation d'un distributeur contenant un conteneur dont l'intérieur est divisé pour définir un volume de stockage (1, 2) et un volume de mesure (5, 6) reliés par l'intermédiaire d'une ouverture de transfert (6), le volume de stockage (1, 2) étant configuré de sorte que, lorsque le distributeur se trouve dans une première orientation, les insectes pré-adultes présents dans le volume de stockage sont guidés vers l'ouverture de transfert pour circuler à travers cette dernière jusqu'à ce que le volume de mesure (5, 6) soit rempli, le volume de mesure ayant un passage de sortie positionné de manière à être inaccessible aux insectes pré-adultes lorsque le distributeur se trouve dans sa première orientation, un mouvement du distributeur vers une seconde orientation conduisant les insectes préadultes se trouvant dans le volume de stockage à quitter l'ouverture de transfert (6) et conduisant les insectes pré-adultes dans le volume de mesure à s'échapper au travers du passage de sortie.

2. Procédé selon la revendication 1, comprenant l'utilisation d'un distributeur dans lequel le volume de stockage (1, 2) est généralement cylindrique et possède une paroi d'extrémité conique (2) aboutissant à un point à une extrémité, le volume de mesure étant défini par un tube de distribution (5) s'étendant essentiellement dans l'axe du volume de stockage, une extrémité du tube se trouvant en contact avec la paroi d'extrémité conique (2) le long d'une ligne entourant le point, et l'autre extrémité de sortie du tube s'étendant à l'extérieur du volume de stockage, l'ouverture de transfert (6) étant formée dans la paroi (5) du tube à son extrémité se trouvant en contact avec la paroi d'extrémité conique (2) du volume de stockage pour constituer un accès à partir du volume de stockage vers le lumen du tube.

3. Procédé selon la revendication 2, comprenant l'utilisation d'un distributeur dans lequel l'ouverture de transfert (6) comprend une découpe à une extrémité du tube de distribution (5).

4. Procédé selon la revendication 3, comprenant l'utilisation d'un distributeur dans lequel l'ouverture de transfert (6) comprend deux découpes (6) diamètralement opposées.

5. Procédé selon la revendication 4, comprenant l'utilisation d'un distributeur dans lequel l'extrémité de sortie du tube de distribution (5) éloignée de la paroi d'extrémité conique (2) du volume de stockage se termine dans un plan incliné par rapport à l'axe longitudinal du volume de stockage et paralléle à l'axe passant par les découpes (6) opposées.

6. Procédé selon la revendication 5, comprenant l'utilisation d'un distributeur dans lequel l'extrémité de sortie du tube de distribution (5) est biseautée.

7. Procédé selon la revendication 5, comprenant l'utilisation d'un distributeur dans lequel l'extrémité de sortie du tube de distribution (5) est inclinée par rapport à l'axe du volume de stockage (1).
